# EUROPEAN PATENT SPECIFICATION

(11) **EP 3 562 419 B1**
(45) Date of publication and mention of the grant of the patent: **09.02.2022**
(21) Application number: 18757984.2
(22) Date of filing: 21.02.2018
(51) Int. Cl.: A61B 18/02, A61M 25/10, A61B 18/00, A61B 90/00, A61M 25/09

(54) **BLOOD VESSEL ISOLATION ABLATION DEVICE**
BLUTGEFÄSSISOLATIONABLATIONSVORRICHTUNG
DISPOSITIF D'ISOLEMENT ET D'ABLATION DE VAISSEAU SANGUIN

(30) Priority: 21.02.2017 US 201762461390 P
(43) Date of publication of application: 06.11.2019
(73) Proprietor: St. Jude Medical, Cardiology Division, Inc., St. Paul, MN 55117 (US)
(72) Inventor: DAHLEN, Travis, Forest Lake, Minnesota 55025 (US); TEGG, Troy, Elk River, Minnesota 55330 (US)
(74) Representative: Kramer Barske Schmidtchen Patentanwälte PartG mbB
(86) International application number: PCT/US2018/018953
(87) International publication number: WO 2018/156580

(56) References cited:
- US-A- 5 766 151
- US-A1- 2006 135 953
- US-A1- 2011 144 637
- US-A1- 2011 144 637
- US-A1- 2014 330 262
- US-A1- 2015 320 472
- US-B1- 6 500 174
- US-B2- 9 314 300
- US-B2- 9 351 783

## Description

### BACKGROUND

US 2014/0330262 A1 discloses a diagnostic guidewire for cryoablation sensing and pressure monitoring. US 2011/0144637 A1 discloses vein occlusion devices and methods for catheter-based ablation. US 9314300 B2 relates to systems for assessing the efficacy of a renal denervation procedure intraoperatively. US 5766151 A relates to a catheter device for use in heart surgery.

The instant disclosure relates generally to tissue ablation. In particular, the instant disclosure relates to a medical device for use in creating a circumferential lesion about a blood vessel, such as in pulmonary vein (PV) isolation procedures.

In the normal heart, contraction and relaxation of the heart muscle (myocardium) takes place in an organized fashion as electro-chemical signals pass sequentially through the myocardium from the sinoatrial (SA) node, which comprises a bundle of unique cells disposed in the wall of the right atrium, to the atrioventricular (AV) node and then a long a well-defined route, which includes the His-Purkinje system, into the left and right ventricles. Sometimes, however, abnormal rhythms occur in the heart, which are referred to generally as arrhythmias.

Certain arrhythmias, referred to as atrial arrhythmias, occur in the atria. Three of the most common atrial arrhythmias are ectopic atrial tachycardia, atrial fibrillation (AF), and atrial flutter. AF can result in significant patient discomfort and even death because of a number of associated problems, including: irregular heart rate, which causes patient discomfort and anxiety; loss of synchronous atrioventricular contractions, which compromises cardiac hemodynamics, resulting in varying levels of congestive heart failure; and stasis of blood flow, which increases the likelihood of thromboembolism, a leading cause of stroke.

One common medical procedure for the treatment of certain types of cardiac arrhythmia, specifically including AF, is catheter ablation. In many ablation procedures, energy, such as radiofrequency (RF) or high intensity focused ultrasound (HIFU) energy, is delivered to cardiac tissue in order to heat the tissue and create a permanent scar or lesion that is electrically inactive.

It is known that, in some instances, stray electrical signals find a pathway down the pulmonary veins and into the left atrium of the heart. In these instances, it may be advantageous to produce a circumferential lesion at or near the ostium of one or more of the pulmonary veins. Desirably, such a circumferential lesion would electrically isolate the pulmonary vein from the left atrium, completely blocking stray signals from traveling down the pulmonary vein and into the left atrium. Thus, such procedures are known as PV isolation procedures.

PV isolation procedures are often carried out cryogenically. More particularly, PV isolation procedures are often carried out using a cryoballoon, such as found on the Arctic Front Advance^{™} Cardiac CryoAblation Catheter from Medtronic.

In a cryoballoon procedure, the practitioner should ensure that the cryoballoon is securely in contact with the PV wall, as gaps between the balloon and the vessel wall can complicate lesion creation. It is known to verify such contact by injecting a contrast medium which can be detected via fluoroscopy. Yet, the increased fluoroscopy dosage is not as desirable.

### BRIEF SUMMARY

The invention is as defined by independent claim 1. Dependent claims 2-11 disclose exemplary embodiments. Disclosed herein is an apparatus for use in ablation procedures, such as cryoablation procedures. The apparatus includes: a ablation catheter *(e.g.,* a cryoablation catheter) including: an elongate body having a lumen, the elongate body including a distal region; and a balloon positioned about the distal region and defining an interior in communication with the lumen, wherein the balloon is expandable outwardly from an outer surface of the elongate body; and a pressure sensor (*e*.*g*., an optical pressure sensor) positioned distally of the balloon. According to aspects of the disclosure, the apparatus also includes an electrophysiology catheter *(e.g.,* a circular mapping catheter) dimensioned for insertion through the lumen, with the pressure sensor including at least one pressure sensor positioned on the distal region of the electrophysiology catheter and the distal region of the ablation catheter. According to one embodiment, a first pressure sensor is positioned on the distal region of the electrophysiology catheter and a second pressure sensor is positioned on the distal region of the ablation catheter.

The apparatus can also include a guidewire over which the ablation catheter can be advanced, and the pressure sensor can include at least one pressure sensor positioned on the distal region of the guidewire and the distal region of the ablation catheter. According to one embodiment, a first pressure sensor is positioned on the distal region of the guidewire and a second pressure sensor is positioned on the distal region of the ablation catheter.

In embodiments disclosed herein, a pressure sensor can be positioned on the elongate body proximally of the balloon.

According to the invention, disclosed herein is a system for use in cryoablation procedures, including: a first device; a second device including: an elongate body having a lumen dimensioned to receive the first device therethrough, the elongate body including a distal region; and a balloon positioned about the distal region and defining an interior in communication with the lumen, wherein the balloon is expandable outwardly from an outer surface of the elongate body; and at least one pressure sensor *(e.g.,* an optical pressure sensor) positioned on the distal region of the second device and a distal region of the first device. The first device can be a guidewire, an electrophysiology catheter, or the like.

According to the invention, the at least one pressure sensor includes a first pressure sensor positioned on the distal region of the first device extending distally of the second device when received in the lumen and a second pressure sensor positioned on the distal region of the second device distally of the balloon.

It is also contemplated that the system can include at least one pressure sensor positioned on the second device proximally of the balloon.

The instant disclosure also provides a method not according to the invention of performing a pulmonary vein isolation. The method includes: introducing an ablation catheter (*e.g.,* a cryoablation catheter) into a left atrium of a heart, the ablation catheter including a balloon; inflating the balloon; advancing the balloon into contact with a wall of a pulmonary vein; verifying that the balloon is occluding the pulmonary vein by measuring a pressure within the pulmonary vein; and delivering an ablation fluid to the balloon to form a circumferential lesion about the pulmonary vein, such as by cooling or heating the cryoballoon. The pressure within the pulmonary vein can be measured by using a pressure sensor mounted to the ablation catheter distal of the balloon.

According to aspects of the disclosure, the step of introducing an ablation catheter into a left atrium of a heart includes introducing the ablation catheter into the left atrium of the heart over a guidewire; and the step of measuring the pressure within the pulmonary vein includes measuring the pressure within the pulmonary vein using a pressure sensor mounted to a region of the guidewire that remains distal of the balloon after introducing the ablation catheter into the left atrium of the heart over the guidewire.

In other aspects of the disclosure, the method can also include introducing an electrophysiology catheter into the pulmonary vein through a lumen in the ablation catheter, and measuring the pressure within the pulmonary vein can include measuring the pressure within the pulmonary vein using a pressure sensor mounted to a region of the electrophysiology catheter that extends distally of the ablation catheter.

The step of verifying that the balloon is occluding the pulmonary vein can include: measuring a pressure within the pulmonary vein using a first pressure sensor positioned distal of the balloon; measuring a pressure within the left atrium using a second pressure sensor positioned proximal of the balloon; and comparing the pressure within the pulmonary vein to the pressure within the left atrium.

In yet another example not according to the invention, the instant disclosure relates to a method of performing a pulmonary vein isolation that includes: verifying occlusion of a pulmonary vein by a balloon utilizing a pressure within the pulmonary vein; and delivering ablation therapy to the pulmonary vein after verifying occlusion of the pulmonary vein. For example, cryoablation therapy can be delivered to the pulmonary vein after verifying occlusion thereof.

The foregoing and other aspects, features, details, utilities, and advantages of the present invention will be apparent from reading the following description and claims, and from reviewing the accompanying drawings.

### BRIEF DESCRIPTION OF THE DRAWINGS

Figure 1 depicts a kit for use in a cryoablation procedure.
Figure 2 depicts details of a cryoablation catheter according to aspects of the instant disclosure.
Figure 3 depicts details of an electrophysiology catheter according to aspects of the instant disclosure.
Figures 4A and 4B depict the conduct of a pulmonary vein isolation procedure.
Figures 5A and 5B depict pressure waveforms within the pulmonary vein during a pulmonary vein isolation procedure.

### DETAILED DESCRIPTION

The present disclosure provides medical devices, such as catheters, suitable for use in creating an ablation lesion about the circumference of a blood vessel. Such a lesion is referred to herein as a "circumferential lesion," and includes both closed-ended lesions (*e.g.,* a circular lesion that loops around the vessel wall a single time) and open-ended lesions (*e.g.,* a helical lesion that loops around the vessel wall multiple times). In particular, the devices disclosed herein allow a practitioner to verify occlusion of the blood vessel, and therefore good contact between the device and the vessel, prior to cryoablation without requiring the introduction of contrast medium.

For purposes of illustration, certain embodiments will be described in the context of a pulmonary vein isolation ("PVI") cryoablation procedure. It should be understood, however, that the teachings herein can be applied to good advantage in other contexts. For example, the teachings herein are applicable to various balloon-based PVI procedures where direct contact with the vessel wall is required, such as procedures that utilize radiofrequency ("RF") energy to create ablation lesions.

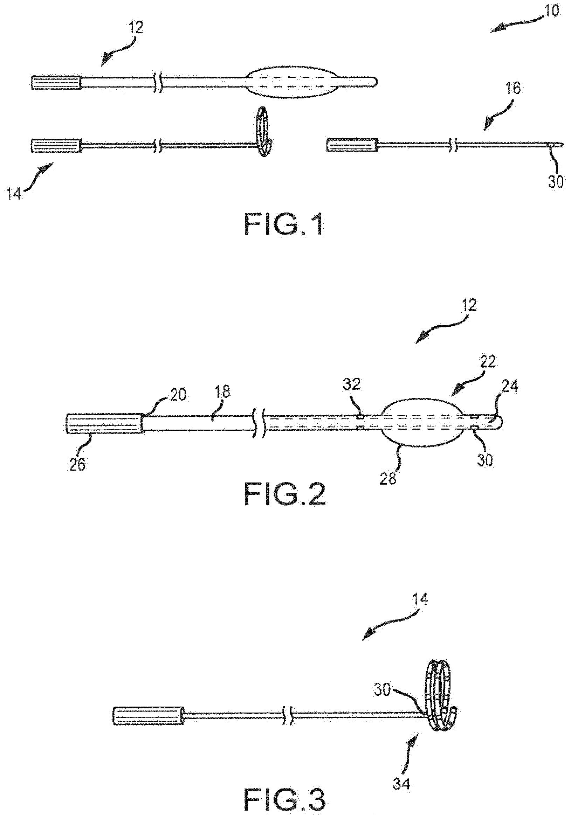

Figure 1 depicts apparatus 10 for use in cryoablation procedures. As shown in Figure 1, apparatus 10 generally includes a cryoablation catheter 12, an electrophysiology catheter 14, and a guidewire 16 over which cryoablation catheter 12 can be advanced through a patient's vasculature. Apparatus 10 can also include devices to facilitate a transseptal approach to the left atrium, such as an introducer and a transseptal puncture needle. Those of ordinary skill in the art will have a basic familiarity with the foregoing components of apparatus 10, such that they will be described herein only to the extent necessary to understand the instant disclosure.

As shown in Figure 2, cryoablation catheter 12 generally includes an elongate body 18 having a proximal end 20 and a distal region 22. Body 18 includes one or more lumens 24 (shown in phantom in Figure 2). Although only a single lumen 24 is depicted in Figure 2 for purposes of illustration, it should be understood that any number and configuration of lumens 24 can be used without departing from the scope of the instant teachings. As the person of ordinary skill in the art will appreciate, body 18 can also contain electrical interconnect wires, pull-wires, and the like.

Proximal end 20 of body 18 is attached to a catheter control handle 26. Catheter control handle 26 can include, for example, an actuator coupled to suitable structure (*e*.*g*., pull wires and/or pull rings) within body 18 in order to effect the deflection of distal region 22 in one or more bending planes. It can also include electrical power, electrical signal, and/or fluidic connections (*e*.*g*., connection to a source of cryogenic fluid).

A balloon 28 is positioned about distal region 22. The interior of balloon 28 is in communication with lumen 24, for example through one or more vias. Thus, a cryogenic fluid can be provided from a fluid source, through lumen 24, in order to expand balloon 28 outwardly from an outer surface of body 18 as well as to cryogenically ablate adjacent tissue.

Figure 3 depicts electrophysiology catheter 14 in further detail. As shown in Figure 3, electrophysiology catheter 14 can be a multi-electrode circular mapping catheter similar to the Inquiry^{™} Optima^{™} Diagnostic Catheter of St. Jude Medical, Inc. Electrophysiology catheter 14 is dimensioned to fit through lumen 24 of cryoablation catheter 12 as discussed below.

As shown in Figures 2 and 3, one or more pressure sensors 30 are mounted on cryoablation catheter 12 and electrophysiology catheter 14. Pressure sensor 30 is mounted on cryoablation catheter 12 distally of balloon 28. Thus, when cryoablation catheter 12 is placed for a PVI procedure, pressure sensor 30 can measure the pressure within the pulmonary vein.

Additionally, pressure sensor 30 is mounted on the distal region 34 of electrophysiology catheter 14. Although this pressure sensor 30 is shown as being mounted to the relatively straight portion of the distal region 34 of electrophysiology catheter 14, it could also be mounted within the looped portion of the distal region 34 of electrophysiology catheter 14 without departing from the scope of the instant teachings. In general, however, positioning pressure sensor 30 on the distal region 34 of electrophysiology catheter 14 will allow it to reside within the pulmonary vein when electrophysiology catheter 14 is advanced through lumen 24 of cryoablation catheter 12 as described below.

It is also contemplated to include one or more pressure sensors 32 on cryoablation catheter 12 positioned proximally of balloon 28. This allows for differential pressure readings both inside and outside the pulmonary vein.

Various types of sensors can be used as pressure sensor(s) 30 and 32. In some embodiments, for example, pressure sensor(s) 30 and/or 32 are optical pressure sensors, such as offered by the Technobis group. In other embodiments, pressure sensor(s) 30 and/or 32 can be printed pressure sensors, pressure-sensitive conductive composite ("PSCC") sensors, strain gauges, pressure sensitive capacitive elements residing within resonant LC circuits, and the like.

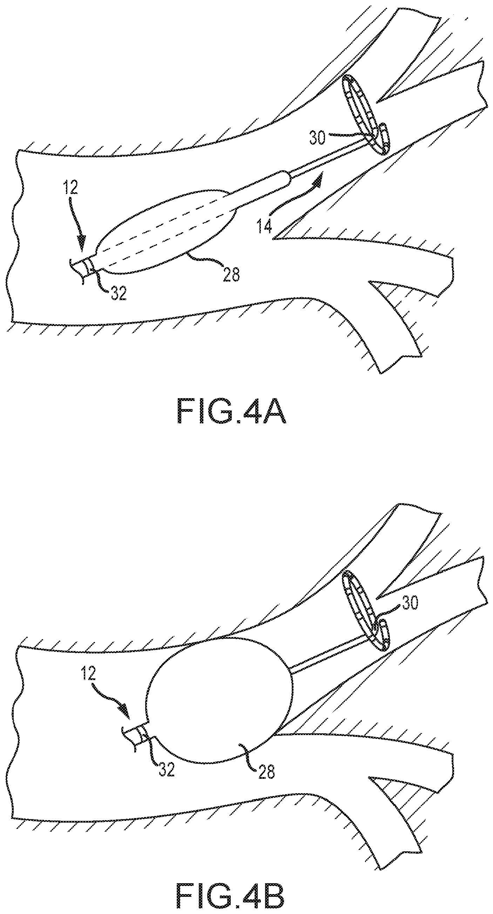

In use, cryoablation catheter 12 is introduced into the left atrium of a patient's heart, for example using a transseptal approach that will be familiar to those of ordinary skill in the art. In the embodiment shown in Figure 4A, cryoablation catheter 12 is introduced over electrophysiology catheter 14. It should be understood, however, that it could also be introduced over guidewire 16 (for example, where the practitioner does not wish to map the pulmonary vein during the procedure).

Balloon 28 can then be inflated and advanced into contact with the wall of a pulmonary vein as shown in Figure 4B. As discussed above, it is desirable to ensure that the pulmonary vein is completely occluded by balloon 28 in order to facilitate the creation of a circumferential lesion about the pulmonary vein. Thus, the pressure within the pulmonary vein can be measured using pressure sensor 30 (shown on electrophysiology catheter 14 in Figures 4A and 4B; as described above, however, it is also carried by cryoablation catheter 12).

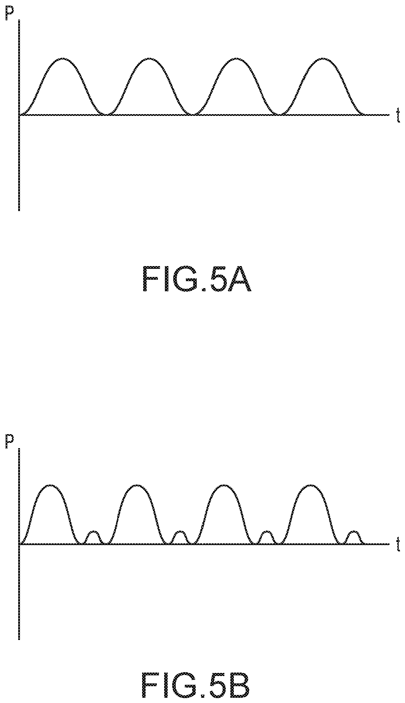

If balloon 28 is completely occluding the pulmonary vein, then the pressure signal from pressure sensor 30 will resemble the trace shown in Figure 5A. The practitioner can then initiate cooling of the cryoballoon to create the circumferential lesion about the pulmonary vein.

If, on the other hand, there is a leak between balloon 28 and the pulmonary vein, then the pressure signal from pressure sensor 30 will resemble the trace shown in Figure 5B. If the practitioner observes a signal resembling Figure 5B, therefore, the practitioner should reposition balloon 28 before cooling the cryoballoon in order to create the circumferential lesion about the pulmonary vein.

In still other embodiments, occlusion van be verified by comparing the pressure measured within the pulmonary vein using pressure sensor 30 to the pressure measured outside the pulmonary vein/in the atrium using pressure sensor 32. St. Jude Medical, Cardiology Division Inc.

Although several embodiments of this invention have been described above with a certain degree of particularity, those skilled in the art could make numerous alterations to the disclosed embodiments without departing from the scope of this invention as defined by the appended claims.

For example, a pressure sensor 30 can also be included within the distal region of guidewire 16 (*see* Figure 1).

All directional references (*e*.*g*., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (*e*.*g*., attached, coupled, connected, and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting. Changes in detail or structure may be made without departing from the scope of the invention as defined in the appended claims.

## Claims

1. A system for use in cryoablation procedures, comprising
a first device (14; 16),
a second device (12) comprising,
an elongate body (18) having a lumen (24) dimensioned to receive the first device (14; 16) therethrough, the elongate body (18) including a distal region (22), and
a balloon (28) positioned about the distal region (22) and defining an interior in communication with the lumen (24), wherein the balloon (28) is expandable outwardly from an outer surface of the elongate body (18), and
a first pressure sensor (30) positioned on the distal region of the first device (14; 16) extending distally of the second device (12) when received in the lumen (24) and
**characterized by**
a second pressure sensor (30) positioned on the distal region (22) of the second device (12) distally of the balloon.

2. The system according to claim 1, wherein the first device comprises a guidewire (16).

3. The system according to claim 1, wherein the first device comprises an electrophysiology catheter (14).

4. The system according to claim 3, wherein the electrophysiology catheter comprises a circular mapping catheter.

5. The system of any one of claims 1 to 4, further comprising at least one pressure sensor (32) positioned on the second device (12) proximally of the balloon (28).

6. The system of claim 5,
wherein the at least one pressure sensor (32) positioned on the elongate body (18) proximally of the balloon (28).

7. The system of any one of claims 1 to 5, wherein
the second device is an ablation catheter (12) comprising
the elongate body (18), and
the balloon (28).

8. The system of claim 7, wherein the first device comprises an electrophysiology catheter (14) dimensioned for insertion through the lumen, and wherein the first pressure sensor (30) is positioned on the distal region of the electrophysiology catheter (14) and the second pressure sensor (30) is positioned on the distal region of the ablation catheter (12).

9. The system according to claim 8, wherein the electrophysiology catheter comprises a circular mapping catheter.

10. The system according to claim 7,
wherein the first device comprises a guidewire (16) over which the ablation catheter (12) can be advanced, and wherein the first pressure sensor (30) is positioned on the distal region of the guidewire (16) and the second pressure sensor (30) is positioned on the distal region of the ablation catheter (12).

11. The system of any one of claims 1 to 10, wherein the pressure sensors comprise an optical pressure sensor.

## Patentansprüche

1. System zur Verwendung bei Kryoablationsverfahren, mit
einer ersten Vorrichtung (14; 16),
einer zweiten Vorrichtung (12), mit
einem länglichen Körper (18), der ein Lumen (24) aufweist, das zum Aufnehmen der ersten Vorrichtung (14; 16) dort hindurch dimensioniert ist, bei dem der längliche Körper (18) einen distalen Bereich (22) aufweist, und
einem Ballon (28), der um den distalen Bereich (22) positioniert ist und einen Innenraum in kommunizierender Verbindung mit dem Lumen (24) definiert, bei dem der Ballon (28) nach außen von einer äußeren Oberfläche des länglichen Körpers (18) ausdehnbar ist, und
einem ersten Drucksensor (30), der an dem distalen Bereich der ersten Vorrichtung (14; 16) positioniert ist, der sich distal von der zweiten Vorrichtung (12) erstreckt, wenn in dem Lumen (24) aufgenommen, und **gekennzeichnet durch**
einen zweiten Drucksensor (30), der an dem distalen Bereich (22) der zweiten Vorrichtung (12) distal des Ballons positioniert ist.

2. System nach Anspruch 1, bei dem die erste Vorrichtung einen Führungsdraht (16) aufweist.

3. System nach Anspruch 1, bei dem erste Vorrichtung einen elektrophysiologischen Katheter (14) aufweist.

4. System nach Anspruch 3, bei dem der elektrophysiologische Katheter einen kreisförmigen Abbildungskatheter aufweist.

5. System nach einem der Ansprüche 1 bis 4, ferner mit zumindest einem Drucksensor (32), der an der zweiten Vorrichtung (12) proximal des Ballons (28) positioniert ist.

6. System nach Anspruch 5, bei dem der zumindest eine Drucksensor (32) an dem länglichen Körper (18) proximal des Ballons (28) positioniert ist.

7. System nach einem der Ansprüche 1 bis 5, bei dem
zweite Vorrichtung ein Ablationskatheter (12) ist, mit
dem länglichen Körper (18) und
dem Ballon (28).

8. System nach Anspruch 7, bei dem die erste Vorrichtung einen elektrophysiologischen Katheter (14) aufweist, der zum Einführen durch das Lumen dimensioniert ist, und bei dem der erste Drucksensor (30) an dem distalen Bereich des elektrophysiologischen Katheters (14) positioniert ist, und der zweite Drucksensor (30) an dem distalen Bereich des Ablationskatheters (12) positioniert ist.

9. System nach Anspruch 8, bei dem der elektrophysiologische Katheter einen kreisförmigen Abbildungskatheter aufweist.

10. System nach Anspruch 7, bei dem die erste Vorrichtung einen Führungsdraht (16) aufweist, über welchen der Ablationskatheter (12) vorgerückt werden kann, und bei dem der erste Drucksensor (30) an dem distalen Bereich des Führungsdrahts (16) positioniert ist, und der zweite Drucksensor (30) an dem distalen Bereich des Ablationskatheters (12) positioniert ist.

11. System nach einem der Ansprüche 1 bis 10, bei dem die Drucksensoren einen optischen Drucksensor aufweisen.

## Revendications

1. Système destiné à être utilisé dans des procédures de cryoablation, comprenant
un premier dispositif (14 ; 16),
un second dispositif (12) comprenant
un corps allongé (18) ayant une lumière (24) dimensionnée pour recevoir le premier dispositif (14 ; 16) à travers celle-ci, le corps allongé (18) comprenant une région distale (22), et
un ballon (28) positionné autour de la région distale (22) et définissant un intérieur en communication avec la lumière (24), dans lequel le ballon (28) est extensible vers l'extérieur depuis une surface extérieure du corps allongé (18), et un premier capteur de pression (30) positionné sur la région distale du premier dispositif (14 ; 16) s'étendant de manière distale par rapport au second dispositif (12) lorsqu'il est reçu dans la lumière (24) et
**caractérisé par**
un second capteur de pression (30) positionné sur la région distale (22) du second dispositif (12) en position distale par rapport au ballon.

2. Système selon la revendication 1, dans lequel le premier dispositif comprend un fil de guidage (16).

3. Système selon la revendication 1, dans lequel le premier dispositif comprend un cathéter d'électrophysiologie ( 14).

4. Système selon la revendication 3, dans lequel le cathéter d'électrophysiologie comprend un cathéter de cartographie circulaire.

5. Système selon l'une quelconque des revendications 1 à 4, comprenant en outre au moins un capteur de pression (32) positionné sur le second dispositif (12) de manière proximale par rapport au ballon (28).

6. Système selon la revendication 5,
dans lequel ledit au moins un capteur de pression (32) est positionné sur le corps allongé (18) à proximité du ballon (28).

7. Système selon l'une quelconque des revendications 1 à 5, dans lequel le second dispositif est un cathéter d'ablation (12) comprenant
le corps allongé (18), et
le ballonnet (28).

8. Système selon la revendication 7, dans lequel le premier dispositif comprend un cathéter d'électrophysiologie (14) dimensionné pour être inséré dans la lumière, et dans lequel le premier capteur de pression (30) est positionné sur la région distale du cathéter d'électrophysiologie (14) et le second capteur de pression (30) est positionné sur la région distale du cathéter d'ablation (12).

9. Système selon la revendication 8, dans lequel le cathéter d'électrophysiologie comprend un cathéter de cartographie circulaire.

10. Système selon la revendication 7,
dans lequel le premier dispositif comprend un fil de guidage (16) sur lequel le cathéter d'ablation (12) peut être avancé, et dans lequel le premier capteur de pression (30) est positionné sur la région distale du fil de guidage (16) et le second capteur de pression (30) est positionné sur la région distale du cathéter d'ablation (12).

11. Système selon l'une quelconque des revendications 1 à 10, dans lequel les capteurs de pression comprennent un capteur de pression optique.
